Europäisches Patentamt

European Patent Office

Office européen des brevets

(11) Publication number: **0 215 388 B1**

## EUROPEAN PATENT SPECIFICATION

(45) Date of publication of patent specification: **04.12.91** (51) Int. Cl.⁵: **C12N 15/77**

(21) Application number: **86112251.3**

(22) Date of filing: **04.09.86**

(54) Plasmid vector and a method for regulation of gene expression using the same.

(30) Priority: **06.09.85 JP 197277/85**
**13.06.86 JP 137833/86**

(43) Date of publication of application:
**25.03.87 Bulletin 87/13**

(45) Publication of the grant of the patent:
**04.12.91 Bulletin 91/49**

(84) Designated Contracting States:
**DE FR GB**

(56) References cited:
**EP-A- 0 063 763**
**EP-A- 0 082 485**
**EP-A- 0 093 611**
**EP-A- 0 158 940**
**EP-A- 0 169 377**

(73) Proprietor: **AJINOMOTO CO., INC.**
**5-8, Kyobashi 1-chome, Chuo-ku**
**Tokyo 104(JP)**

(72) Inventor: **Morinaga, Yasushi Central Research**
**Laboratories**
**Ajinomoto Co.,Inc. 1-1, Suzuki-cho Kawasaki-**
**ku**
**Kawasaki-shi Kanagawa-ken(JP)**
Inventor: **Tsuchiya, Makoto Central Research**
**Laboratories**
**Ajinomoto Co.,Inc. 1-1, Suzuki-cho Kawasaki-**
**ku**
**Kawasaki-shi Kanagawa-ken(JP)**

(74) Representative: **Strehl, Schübel-Hopf, Groen-**
**ing**
**Maximilianstrasse 54 Postfach 22 14 55**
**W-8000 München 22(DE)**

## Description

BACKGROUND OF THE INVENTION

Field of the Invention

The present invention relates to a method for controlling phenotypic gene expression of Coryneform bacteria.

Description of the Prior Art

Coryneform bacteria are microorganisms which are industrially important. Many Coryneform bacteria which produce large quantities of L-glutamic acid are known, including their mutants producing amino acids such as lysine, etc., and purine nucleotides such as inosinic acid, etc.

On the other hand, breeding and improvement of microorganisms for industrial use utilizing recombinant DNA techniques have been recently attempted in, for example, Escherichia coli. : With respect to Coryneform bacteria, some vectors are known as growing in these microorganisms as a host and expressing chemical resistance as a marker (e.g., Published European Patent Application No. 93611), but no method for artificially regulating inserted foreign genes has been discovered. Therefore, it has been difficult to regulate gene expression artificially while expressing foreign genes using Coryneform bacteria as a host.

SUMMARY OF THE INVENTION

Accordingly, it is an object of the present invention to provide a plasmid vector capable of regulating foreign gene expression in Coryneform bacterial cells.

It is a further object of this invention to provide a method for regulating the expression of foreign genes in Coryneform bacterial cells.

These and other objects of the present invention as will hereinafter become more readily apparent have been accomplished by providing a plasmid vector capable of replicating in a Coryneform bacterial cell having a base sequence (a) functioning as a promotor in the Coryneform bacterium, a base sequence (b) functioning as an operator downstream from the base sequence (a), a base sequence (c) functioning as a site for ribosome binding in a Coryneform bacterial cell, a base sequence (d) functioning as a translation initiation site, and a gene to be expressed, which is directly ligated with base sequence (d) and bears a gene coding for a repressor protein capable of binding to the base sequence (b) functioning as an operator.

The invention further comprises a method for regulating gene expression in Coryneform bacterial cells, which involves using a Coryneform bacterial cell having a plasmid vector comprising base sequences (a)-(d) and the gene to be expressed which is directly ligated with base sequence (d) and bearing a gene coding for a repressor protein.

BRIEF DESCRIPTION OF THE DRAWINGS

A more complete appreciation of the invention, and many of the attendant advantages thereof will be readily obtained as the invention becomes better understood by reference to the following detailed description when considered in connection with the accompanying drawings, wherein:

FIGURE 1 is a drawing explaining the procedure for constructing a promoter-detecting vector pEB 001. The abbreviations have the following meanings:

P : Pst I
C : Cla I
H : Hind III
B : BamH I
Km$^r$ : kanamycin-resistant gene
Cm$^s$ : structural gene region of chloramphenicol-resistant gene

FIGURE 2 is a drawing explaining the procedure for constructing a promoter-detecting vector pEB 003. The abbreviations have the following meanings:

P : Pst I
C : Cla I
H : Hind III

2

B    : BamH I
S    : Sal I
Km$^r$   : kanamycin-resistant gene
Cm$^s$  : structural gene region of chloramphenicol-resistant gene

FIGURE 3 shows a structure of DNA fragment C used for the formation of a promoter-detecting vector pEB 003.

FIGURE 4 is a drawing explaining the procedure for constructing a gene expression-regulating vector pEC 701.

FIGURE 5 is a drawing explaining the procedure for constructing a gene expression-regulating vector pEC 702.

FIGURE 6 is a drawing explaining the procedure for constructing a gene expression-regulating vector pEC 801.

FIGURE 7 is a drawing explaining the procedure for constructing a gene expression-regulating vector pEC 901.

FIGURE 8 shows the structure of trimethoprim-resistant vector pAJ 228.

FIGURE 9 is a drawing explaining the procedure for constructing a gene expression-regulating vector pEC 830.


DESCRIPTION OF THE PREFERRED EMBODIMENTS

Coryneform bacteria are aerobic, gram-positive rods, non-acid fast and described in Bergey's Manual of Determinative Bacteriology, 8th edition, page 599 (1974). Examples of wild strains of Coryneform bacteria which can be utilized as host bacteria in the present invention include the following:
Brevibacterium divaricatum ATCC 14020,
Brevibacterium saccharolyticum ATCC 14066,
Brevibacterium immariophilum ATCC 14068,
Brevibacterium lactofermentum ATCC 13869,
Brevibacterium roseum ATCC 13825,
Brevibacterium flavum ATCC 13826,
Brevibacterium thiogenitalis ATCC 19240,
Corynebacterium acetoacidophilum ATCC 13870,
Corynebacterium acetoglutamicum ATCC 15806,
Corynebacterium callunae ATCC 15991,
Corynebacterium glutamicum ATCC 13032, 13060,
Corynebacterium lilium ATCC 15990,
Corynebacterium melassecola ATCC 17965, and
Microbacterium ammoniaphilum ATCC 15354.

Coryneform bacteria include mutants which are derived from these glutamic acid-producing bacteria or have lost glutamic acid productivity, and mutants which produce amino acids such as lysine, arginine, etc., purine nucleosides such as inosine, etc., purine nucleotides such as inosine-5'-mono-phosphate, etc. and other products. Any plasmid that proliferates in Coryneform bacterial cells can be used as the plasmid capable of replicating in Coryneform bacterial cells. Examples of wild strains of such a plasmid include the following:

    (1) pAM 330:    cf. Published Unexamined Japanese Patent Application 67699/83
    (2) pHM 1519:  cf. Published Unexamined Japanese Patent Application 77895/83
    (3) pCG 1:     cf. Published Unexamined Japanese Patent Application 134500/82
    (4) pCG 2:     cf. Published Unexamined Japanese Patent Application 35197/83
    (5) pCG 4:     cf. Published Unexamined Japanese Patent Application 183799/82.

As the plasmid capable of replicating in Coryneform bacterial cells, it is sufficient to have contained therein an initiation site for replication of these wild plasmids. Therefore, there is no particular problem even though the vector contains DNA's other than the initiation site for replication.

Any sequence can be used as the base sequence (a) functioning as a promoter as long as it is a base sequence functioning as a promoter in Coryneform bacterial cells. Of course, the base sequence can be a base sequence of a promoter derived from Coryneform bacteria and base sequences of foreign promoters such as base sequences of various promoters derived from Escherichia coli (D.K. Hawley and W. McClure, Nucleic Acids Research 11, 2237-2255 (1983)), and others.

The base sequence (b) functioning as an operator can be any base sequence as long as the repressor protein can be bound thereto. Of course, the base sequence (b) includes a base sequence of an operator derived from Coryneform bacteria, base sequences of various operators derived from Escherichia coli, e.g., base sequences of foreign operators such as a lac operator, a trp operator (Kenzo Nakamura, KAGAKU-NO-RYOIKI, 37(5), 349-362 (1983)), a λ operator (Erik Remaut et al, Gene, 15, 81-93 (1983)), an operator of a phosphatase operon (Hideo Shinagawa et al, Journal of Bacteriology, Japan, 40, 211 (1985)), and others. The above-mentioned references are incorporated herein the same as if each were individually reproduced in its entirety at this location.

The base sequence (c) is a base sequence comprising 4 base pairs rich in adenine (A) and guanine (G). In particular, the upstream 2 base pairs are rich in A and the downstream 2 base pairs are rich in G. The distance between the base sequence (d) and the base sequence (c) is 7 base pairs or more but when the number of base pairs is large (exceeds 20 base pairs), the efficiency of desired gene expression declines. A preferred range of base pair distance is 10 to 15 base pairs between base sequence (d) and base sequence (c).

The investigations by the present inventors revealed that in the base sequence (d), ATG or GTG functions as a translation initiation codon in Coryneform bacteria.

The gene to be expressed, which is directly ligated to the base sequence (d), may be any gene derived from microorganisms such as bacteria belonging to the genus Coryneform, bacteria belonging to the genus Streptomyces, yeast belonging to the genus Saccharomyces, bacteria belonging to the genus Escherichia and Bacillus, and so on, genes derived from animals, artifically synthesized genes, and the like. Further, the expression products of the gene to be expressed are enzymes which participate in production of amino acids, glucose, vitamins, etc., improvement in quality of oils and fats, proteins, starch, etc., modification of antibiotics, chemical substances, etc. such as hydrolase, transaminase, decarboxylase, phosphotransferase, etc., as well as immune modulators such as lymphokines, etc., physiologically active proteins such as animal hormones, etc.

As the gene coding for the repressor protein, any gene can be used as long as the protein the gene encodes can bind to the base sequence (b) of the operator used in Coryneform bacteria and the gene has a property such that the binding activity can be artifically controlled. Such a gene includes, of course, a repressor gene derived from Coryneform bacteria, various repressor genes derived from Escherichia coli, e.g., the lac repressor (lac I) (Miller and Reznikoffs, The Operon, Cold Spring Harbor Lab., pp. 31-88 (1978)-), the trp repressor (trp R) (R.L. Kelley and C. Yanofsky, Proc. Natl. Acad, Sci. USA, 79, 3120-3124 (1982)), the temperature-sensitive λ repressor (cI 857) (J.J. Sninsky et al., Gene, 16, 275-286 (1982)), the repressor of the phosphatase operon (H. Shinagawa et al., J. Mol. Biol., 168, 477-488 (1983)), and so on. As means for artificially regulating the binding of these repressor proteins to the operator site, addition of chemicals, regulation of physical conditions such as temperature, and others may be used. For example, the binding of the lac repressor to the lac operator can be inhibited by addition of isopropyl-$\beta$ -thiogalactopyranoside (IPTG); the binding of the trp repressor to the trp operator can be inhibited by addition of indole acrylic acid (IAA); the binding of the temperature-sensitive λ repressor to the λ operator can be inhibited by increasing culture temperature to 37°C or higher; and the binding of the repressor of the phosphatase operon to the operator can be inhibited by lowering phosphate ion concentration in the medium.

By inhibiting the binding between the repressor and the operator by the foregoing methods, the promoter inactivated by the repressor can be activated so that the gene which is initially not expressed can be changed to a state such that it is expressed.

The base sequence (b) functioning as the operator and the gene coding for the repressor protein can be incorporated into the same plasmid; alternatively, they can be independently incorporated into two different plasmids capable of co-existing in the same host and the two plasmids can co-exist in the same host cell. In any, event, it is sufficient that the repressor protein be capable of binding the operator in host cells.

Further, a gene the expression of which confers resistance to an antibiotic, or the like acting as a selection marker is generally inserted in the plasmid(s).

To introduce the obtained plasmid(s) into Coryneform bacterial cells, conventional methods such as the protoplast method, among others, are applicable.

The invention now being generally described, the same will be better understood by reference to certain specific examples which are included herein for purposes of illustration only and are not intended to be limiting of the invention or any embodiment thereof, unless specified.

EXAMPLES

Example 1

Formation of pEB 001

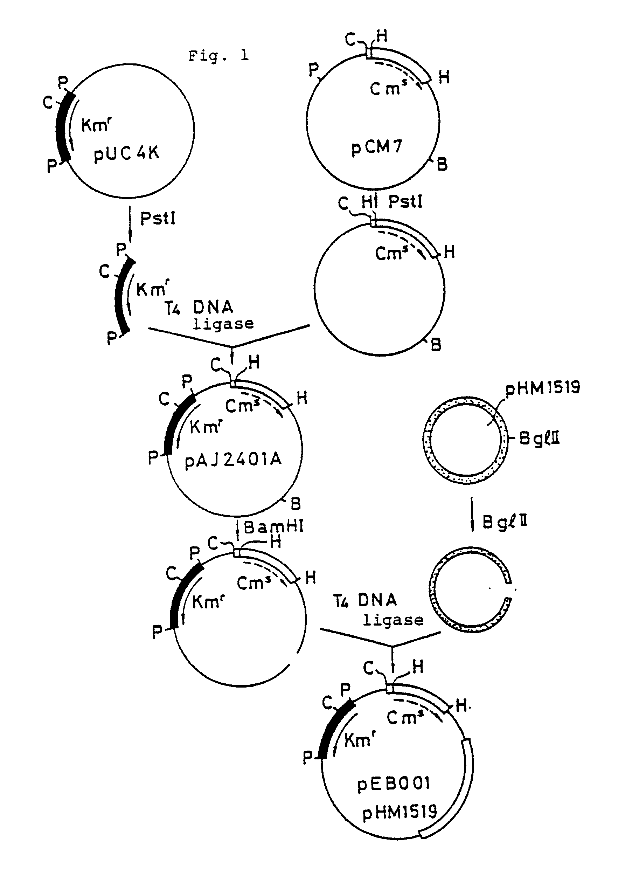

Promoter-probing vector pEB 001 carrying a chloramphenicol-resistant gene which had lost a promoter sequence, being capable of replicating in cells of Coryneform bacteria, expressing kanamycin resistance, and carrying a site cleavable by Cla I and Hind III as a site capable of having inserted therein a promoter was formed by the method shown in FIGURE 1. That is, plasmid pUC 4K (cf. Gene, 19, 259-268 (1982), and J. Mol. Biol., 147, 217-226 (1981)) carrying a kanamycin-resistant gene derived from transposon Tn 903 was first cleaved with Pst I to excise a DNA fragment of about 1.4 kb containing a kanamycin-resistant gene. After mixing with DNA of plasmid pCM 7 (cf. Gene, 20, 305-316 (1982)), containing a structural gene region of a chloramphenicol-resistant gene cleaved with Pst I, ligation was performed using $T_4$ DNA ligase. The obtained recombinant DNA was introduced into Escherichia coli HB101 strain to select a strain having kanamycin resistance. From the separated strain, plasmid DNA was obtained. It was confirmed that the kanamycin-resistant gene had been correctly inserted by the size of the plasmid and the fact that a DNA fragment of about 1.4 Kb could be obtained by cleavage with Pst I. This plasmid was named pAJ 2401A. Next, in order to impart a replication ability in cells of Coryneform bacteria to pAJ 2401A, DNA of plasmid pHM 1519 (cf. Published Unexamined Japanese Patent Application 77895/83) capable of replicating in cells of Coryneform bacteria was cleaved with Bgl II followed by mixing with DNA of pAJ 2401A cleaved with BamH I and ligating using $T_4$ DNA ligase. The obtained recombinant DNA was introduced into Escherichia coli HB101 strain to select a strain having a kanamycin resistance. From the separated stain, plasmid DNA was obtained. It was confirmed that it was the desired plasmid by the size of the plasmid and by lack of cleavage by BamH I and Bgl II. This plasmid was named pEB 001. pEB 001 was introduced into Brevibacterium lactofermentum AJ 12036 (FERM BP-734) and Corynebacterium glutamicum ATCC 13060 and a strain having a kanamycin resistance was selected. From the separated strain, plasmid DNA was obtained. It was confirmed that it was DNA of pEB 001 by the size of the plasmid and cleavage patterns with restriction enzymes such as Pst I, BamH I, etc. From these strains, the strain Brevibacterium lactofermentum, in which a clear band of DNA of pEB 001 had been detected, was deposited as AJ 12177 (FERM-P7942). As above, pEB 001, which is a shuttle vector capable of replicating in Escherichia coli, Brevibacterium lactofermentum and Corynebacterium glutamicum and expresing kanamycin resistance, carries a structural gene region of the chloramphenicol-resistant gene which has lost the promoter part, and carries a Cla I and Hind III cleavage site as a site capable of inserting a promoter.

DNA of pCM 7 used in this example was prepared from Escherichia coli ATCC 37173. DNA of pUC 4K was purchased from Pharmacia Japan Co., Ltd. Further, DNA of pHM 1519 was prepared from Corynebacterium glutamicum ATCC 13058, a strain possessing this DNA.

Formation of pEB 003

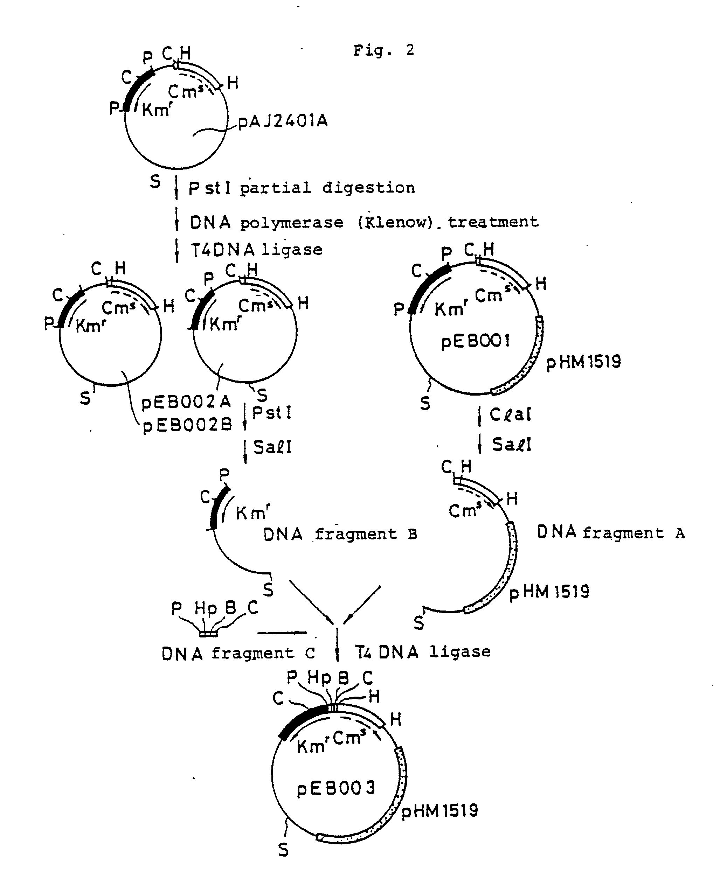

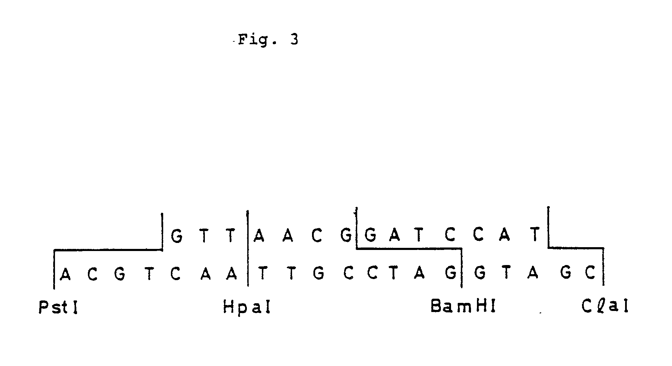

Promoter-detecting vector pEB 003 carrying a structural gene region of a chloramphenicol-resistant gene which had lost a promoter sequence, being capable of replicating in cells of Coryneform bacteria, expressing kanamycin resistance, and carrying a site cleavable with 5 restriction enzymes (Pst I, Hpa I, BamH I, Cla I and Hind III) as a site capable of having a promoter inserted therein was formed by the method shown in FIGURE 2. First, DNA from pAJ 2401A was partly cleaved with Pst I. The DNA fragments formed by cleavage at one site were collected by agarose gel and a single strand portion projecting at both edges of the DNA fragments was removed to produce blunt ends. Then, ligation was performed using $T_4$ DNA ligase. The obtained recombinant DNA was introduced into Escherichia coli HB101 strain and a strain having a kanamycin resistance was selected. From the separated strain, plasmid DNA was obtained, and plasmid DNA cleaved with Pst I at one site was selected. In these plasmids, a plasmid which had lost the Pst I cleavage site at the 5'-side and a plasmid which had the Pst I cleavage site at the 3'-side of the kanamycin-resistant gene were present. The former plasmid was named pEB 002A and the latter was named pEB 002B. Next, DNA of pEB 001 was cleaved with Cla I and Sal I to obtain DNA fragment A containing the structural gene region of the chloramphenicol-resistant gene and a replication initiation site in cells of Coryneform bacteria. On the other hand, pEB 002A was cleaved with Pst I and Sal I to obtain DNA fragment B containing a kanamycin-resistance gene. Further, an oligonucleotide as shown in FIGURE 3 was synthesized according to the phosphite method to prepare DNA fragment C carrying a Pst I cleavage site at the 5'-terminal, a Cla I cleavage site at the 3'-terminal and a Hpa I and BamH I cleavage site therebetween. After mixing DNA fragments A, B and C, they were ligated using $T_4$ DNA ligase. The obtained recombinant DNA was introduced into Brevibacterium lactofermentum AJ 12036 (FERM BP-734) and strains having a kanamycin resistance were selected. From the separated strains, plasmid DNA was obtained. It was confirmed that it was the desired plasmid DNA by the size of the plasmid and the cleavage patterns with restriction enzymes such as Pst I, Hpa I, BamH I, etc. This plasmid was named pEB 003. pEB 003 possesses cleavage sites with 5 restriction enzymes (Pst I, Hpa I, BamH I, Cla I and Hind III) as sites where a promoter may be inserted. Among them, the cleavage site with Pst I, Hpa I and BamH I is the only one in the vector which is extremely useful for insertion of a promoter.

Brevibacterium lactofermentum AJ 12036 (FERM BP-734) to which pEB 003 had been introduced was deposited as AJ 12178 (FERMP 7943).

Incorporation of a tac promoter and a lac operator into pEB 003

DNA of plasmid pDR 540 (cf. Gene, 20, 231 (1982)) (commercially available, purchased from Pharmacia Japan Co., Ltd.) carrying the Escherichia coli tac promoter and lac operator was cleaved with BamH I and Pst I. The obtained DNA fragments of about 1100 base pairs containing the tac promoter and the lac operator were fractionated and purified by agarose gel electrophoresis. After mixing with DNA of pEB 003 cleaved with Pst I and BamH I, ligation was performed using T₄ DNA ligase. The obtained recombinant DNA was introduced into Escherichia coli HB 101 strain and strains having a chloramphenicol resistance were selected. From the separated strains, plasmid DNA was obtained. It was confirmed that it was a plasmid having incorporated therein the Escherichia coli tac promoter as desired, by the size of the plasmid and cleavage patterns with restriction enzymes such as Pst I, BamH I, etc. This plasmid was named pEB 003TA.

Next, pEB 003TA was introduced into Brevibacterium lactofermentum AJ 12036 (FERM BP-734) and strains having a kanamycin resistance were selected. From the separated strains, a plasmid was obtained. It was confirmed that the plasmid was pEB 003TA by the size of the plasmid and cleavage patterns with restriction enzymes such as Pst I, BamH I, etc.

Formation of a gene expression-regulating vector pEC 701

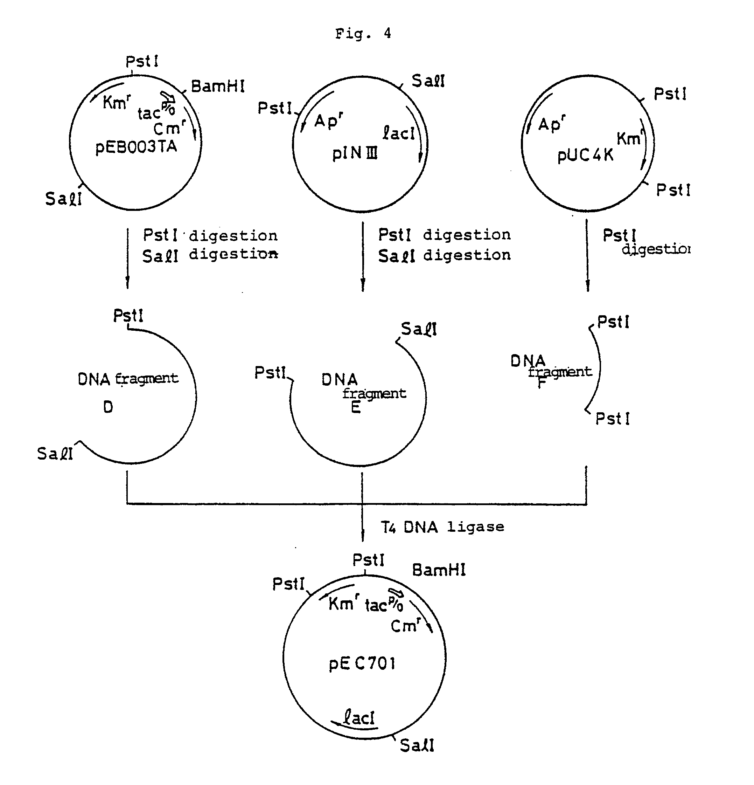

Plasmid pEC 701, capable of regulating expression of a gene ligated downstream from a tac promoter and a lac operator region by the action of a regulating gene (lac repressor) was formed by the method as shown in FIGURE 4.

First, after pEB 003TA (formed as above) was cleaved with Pst I and Sal I, DNA fragment D of about 5.6 kb was recovered from agarose gel. On the other hand, the plasmid pIN III (y. Masui et al., Expermental Manipulation of Gene Expression, Ed. M. Inouye, pp. 15, Academic Press, Inc. 1983) of Escherichia coli was cleaved with Pst I, and Sal I, and DNA fragment E of about 5.6 kb containing the lac repressor (lac I) was recovered from agarose gel. Further, plasmid pUC 4K (Gene, 19, 256-268 (1982)) of Escherichia coli was cleaved with Pst I, and DNA fragment F of about 1.2 kb containing a kanamycin-resistant gene was likewise recovered from agarose gel. The thus obtained 3 DNA fragments D, E and F were mixed in an approximately equal amount. After ligating with T₄ DNA ligase, they were introduced into Brevibacterium lactofermentum AJ 12036 (FERM BP-734) and strains showing a kanamycin resistance were selected. From the separated strains, plasmid DNA of about 12.4 kb composed by ligation of 3 DNA fragments D, E and F was obtained. It was confirmed that it was the desired plasmid by cleavage patterns with restriction enzymes such as Pst I, Sal I, etc. This plasmid was named pEC 701. pEC 701 was again introduced into Brevibacterium lactofermentum AJ 12036 (FERM BP-734) and Corynebacterium glutamicum ATCC 13060, from which strains those showing a kanamycin resistance were selected. From the separated strains, plasmid DNA was obtained. It was confirmed that all DNA was that of pEC 701 by the size of the plasmid and cleavage patterns with restriction enzymes such as Pst I, Sal I, etc.

The thus formed pEC 701 is a shuttle vector carrying a tac promoter, a lac operator and a lactose operon repressor (lac repressor), being capable of replicating in Escherichia coli, Brevibacterium lactofermentum and Corynebacterium glutamicum and expressing a kanamycin resistance. It is also a vector bearing a structural gene region of a chloramphenicol-resistant gene, and is capable of regulating expression of the chloramphenicol-resistant gene by the lac repressor. Further, there are sites which can be cleaved with restriction enzymes such as BamH I, etc. downstream from the lac operator. pEC 701 is also a vector capable of regulating expression of the gene by incorporating a structural gene region free from a promoter of any useful gene, utilizing these sites as cloning sites.

Example 2

Incorporation of a lac promoter and operator into pEB 003

DNA of plasmid pGL 101 (cf. J. Mol. Appl. Genet., 1, 139 (1981)) carrying the Escherichia coli lac UV5 promoter and operator was cleaved with Pst I and Pvu II. The obtained DNA fragments of about 1000 base pairs containing the lac UV5 promoter and operator were fractionated and purified by agarose gel electrophoresis. After mixing with the fragments containing the lac UV promoter and operator and DNA of pEB 003 cleaved with Pst I and Hpa I, ligation was performed using T₄ DNA ligase. The obtained recombinant DNA was introduced into Escherichia coli HB 101 strain and strains having a chloramphenicol resistance were selected. From the separated strains, plasmid DNA was obtained. It was confirmed that it was a plasmid having incorporated therein the Escherichia coli lac UV5 promoter and operator as desired, by the size of the plasmid and cleavage patterns with restriction enzymes such as Pst I, BamH I, etc. This plasmid was named pEB 003LA.

Next, pEB 003LA was introduced into Brevibacterium lactofermentum AJ 12036 (FERM BP-734) and strains having a kanamycin resistance were selected. From the separated strains, a plasmid was obtained. It was confirmed that the plasmid was pEB 003LA by the size of the plasmid and cleavage patterns with restriction enzymes such as Pst I, BamH I, etc.

Formation of a gene expression-regulating vector pEC 702

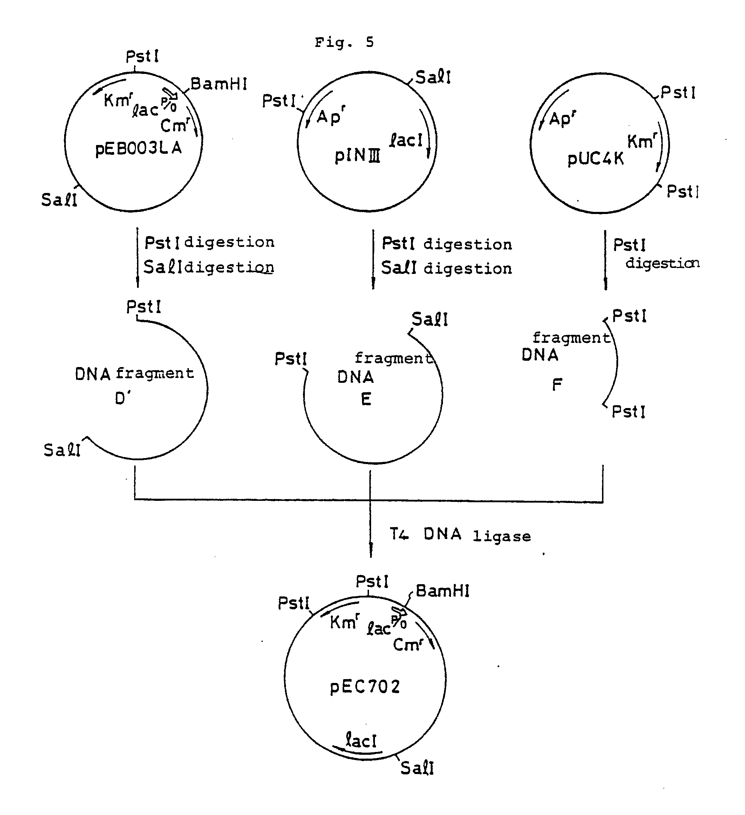

Plasmid pEC 702, capable of regulating expression of a gene ligated downstream from a lac promoter and lac operator region by the action of the lac repressor, was formed by the method shown in FIGURE 5. After pEB 003LA was cleaved with Pst I and Sal I, DNA fragment D' of about 5.5 kb containing the lac promoter and operator was recovered from agarose gel. After ligating the fragment D', the Pst I-Sal I fragment E of about 5.6 kb containing the lac repressor (lac I) excised from plasmid pIN II and the Pst I fragment F of about 1.2 kb containing a kanamycin-resistant gene excised from pUC 4K using T₄ DNA ligase in a manner similar to Example 1, they were introduced into Brevibacterium lactofermentum AJ 12036 (FERM BP-734) and strains showing a kanamycin resistance were selected. From the separated strains, plasmid DNA of about 12.3 kb, composed of 3 ligated DNA fragments D', E and F, was obtained. It was confirmed that it was the desired plasmid by cleavage patterns with restriction enzymes such as Pst I I, Sal I, etc. This plasmid was named pEC 702. pEC 702 was again introduced into Brevibacterium lactofermentum AJ 12036 (FERM BP-734) and Corynebacterium glutamicum ATCC 13060, from which strains those showing a kanamycin resistance were selected. From the separated strains, plasmid DNA was obtained. It was confirmed that all of this DNA was that of pEC 702 by the size of the plasmid and cleavage patterns with restriction enzymes such as Pst I, Sal I, etc.

The thus formed pEC 702 is a shuttle vector carrying a lac promoter, a lac operator and a lac repressor, being capable of replicating in Escherichia coli, Brevibacterium lactofermentum and Coryneform, and expressing a kanamycin resistance. It is also a vector bearing a structural gene region of a chloramphenicol-resistant gene and is capable of regulating expression of the chloramphenicol-resistant gene by the lac repressor. Further, there are sites which may be cleaved by restriction enzymes such as BamH I, etc. downstream from the lac operator. pEC 701 is also a vector capable of regulating expression of the gene by incorporating a structural gene region of any useful gene free from a promoter utilizing these sites as cloning sites.

Example 3

Regulation of gene expression using the gene expression-regulating vectors pEC 701 and pEC 702

Brevibacterium lactofermentum AJ 12036 (FERM-BP 734) harboring pEC 701 or pEC 702, was inoculated on 50 ml of liquid medium of pH 7.2 containing 1% yeast extract, 1% polypeptone, 0.5% NaCl, 0.5% glucose and 10 μg/ml of kanamycin, followed by shake culture at 30° C. After culturing for 3 hours, 0.2 mM of isopropyl-ß-thiogalactopyranoside (IPTG) was supplemented thereto and culture was continued. With passage of time, 5 ml samples of the culture solution were taken and bacteria were collected by centrifugation. After washing, the cells were ground by way of ultrasonic waves and centrifuged at 15,000 r.p.m. for 15 minutes. The supernatant was used as a crude enzyme solution and measured with respect to chloramphenicol acetyltransferase activity. The chloramphenicol acetyltransferase activity was measured by the method of W.V. Shaw, Methods in Enzymology, 43, 737 (1975). Similar runs were also performed on Brevibacterium lactofermentum AJ 12036 having no plasmid, AJ 12036 strain harboring plasmid pEB 003TA having a tac promoter and a lac operator but having no lac repressor, and AJ 12036 strain harboring plasmid pEB 003LA having a lac promoter and a lac operator but having no lac repressor. The results are shown in Table 1. It was verified that by the addition of IPTG, the chloramphenicol acetyltransferase activity was enhanced by about 10 fold with the pEC 701-harboring strain and the pEC 702-harboring strain, respectively, as compared to the case where none is added, and gene expression of chloramphenicol acetyltransferase could be artificially regulated by the addition of IPTG in both the pEC 701-harboring strain and the pEC 702-harboring strain. On the other hand, in both the pEB 003TA-harboring strain and the pEB 003LA-harboring strain, high activity was noted irrespective of the presence or absence of IPTG and regulation of gene expression was impossible.

## Table 1

Regulation of chloramphenicol acetyltransferase gene in
Brevibacterium lactofermentum AJ 12036 using the gene-
expression regulating plasmids pEC 701 and pEC 702

| Plasmid | IPTG | Activity of Chloramphenicol Acetyltransferase | | |
| --- | --- | --- | --- | --- |
| | | 0 hr | 1 hr | 2 hr |
| None | − | 0 | 0 | 0 |
| | + | 0 | 0 | 0 |
| pEB 003TA | − | 470 | 460 | 320 |
| | + | 470 | 460 | 300 |
| pEC 701 | − | 18 | 23 | 28 |
| | + | 18 | 101 | 205 |
| pEB 003LA | − | 168 | 166 | 160 |
| | + | 168 | 165 | 155 |
| pEC 702 | − | 6 | 6 | 5 |
| | + | 6 | 42 | 36 |

Example 4

Incorporation of a trp promoter and a trp operator into pEB 003

DNA of plasmid pDR 720 (cf. Gene, 20 231 (1982)) (commercially available, purchased from Pharmacia Japan Co., Ltd.) carrying the Escherichia coli trp promoter and trp operator was cleaved with Pst I. The obtained DNA fragments of about 1100 base pairs containing the trp promoter and trp operator were fractionated and purified by agarose gel electrophoresis. After mixing the DNA fragments with DNA of pEB 003 cleaved with Pst I, ligation was performed using T4 DNA ligase. The obtained recombinant DNA was introduced into Escherichia coli HB 101 strain and strains having a chloramphenicol resistance were selected. From the separated strains, plasmid DNA was obtained. It was confirmed that it was a plasmid having incorporated therein the Escherichia coli trp promoter and trp operator as described, by the size of the plasmid and cleavage patterns with restriction enzymes such as Pst I, Pvu I, etc. This plasmid was named pEB 003TR.

Next, pEB 003TR was introduced into Brevibacterium lactofermentum AJ 12036 (FERM BP-734) and strains having a kanamycin resistance were selected. From the separated strains, a plasmid was obtained. It was confirmed that the plasmid was pEB 003TR by the size of the plasmid and cleavage patterns with restriction enzymes such as Pst I, Pvu I, etc.

Formation of a gene expression-regulating vector pEC 801

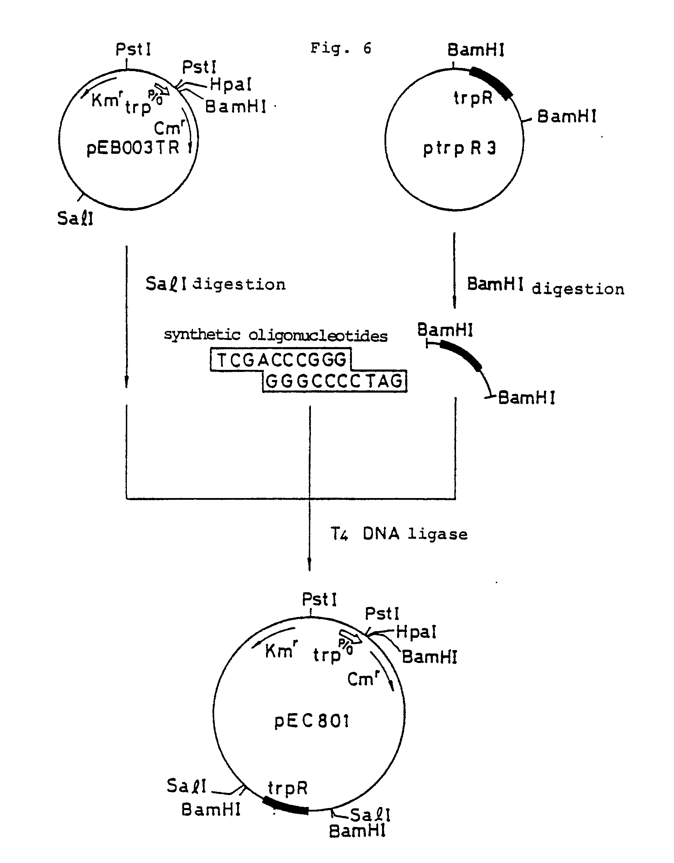

Plasmid pEC 801 capable of regulating expression of a gene ligated downstream from a trp promoter and trp operator region by the action of a regulating gene (trp repressor) was formed by the method shown in FIGURE 6. First, pEB 003TR (formed as above) was cleaved with Sal I. On the other hand, Escherichia coli plasmid ptrp R3 (W. Roeder and R.L. Somerville, Mol. Gene. Genet., 176, 361 (1979)) was cleaved with BamH I and, about 1200 base pairs of DNA fragments containing the trp repressor (trpR) were recovered from agarose gel. The thus obtained two DNA's were ligated with T₄ DNA ligase via a synthesized DNA adaptor synthesized by the phosphite method. Thereafter, they were introduced into Brevibacterium lactofermentum AJ 12036 (FERM BP-734) and strains showing a kanamycin resistance were selected. From the separated strains, plasmid DNA of about 9.7 kb composed by ligation of the above-described 2 DNA fragments was obtained. It was confirmed that it was the desired plasmid by the cleavage pattern with BamH I. This plasmid was named pEC 801. pEC 801 was again introduced into Brevibacterium lactofermentum AJ 12036 (FERM BP-734) and Corynebacterium glutamicum ATCC 13060, from which strains those showing a kanamycin resistance were selected. From the separated strains, plasmid DNA's were obtained. It was confirmed that all of these were DNA of pEC 801 by the size of the plasmid and the cleavage pattern with BamH I.

The thus formed pEC 801 is a shuttle vector carrying a trp promoter, a trp operator and a trp repressor, which is capable of replicating in Escherichia coli, Brevibacterium lactofermentum and Corynebacterium glutamicum and expressing a kanamycin resistance. It is also a vector bearing a structural gene region of a chloramphenicol-resistant gene and is capable of regulating expression of the chloramphenicol-resistant gene by the trp repressor. Further, there are sites which may be cleaved with restriction enzymes such as Pst I, Hpa I, BamH I, etc. downstream from the trp promoter. pEC 701 is also a vector capable of regulating expression of the gene by incorporating a structural gene region free from a promoter, of any useful gene, utilizing these sites as cloning sites.

Regulation of gene expression using the gene express-regulating vector pEC 801

Brevibacterium lactofermentum AJ 12036 (FERM-BP 734) harboring pEC 801 was inoculated on 50 ml of liquid medium of pH 7.2 containing 1% yeast extract, 1% polypeptone, 0.5% NaCl, 0.5% glucose and 10 μg/ml of kanamycin followed by shake culture at 30°C. After culturing for 3 hours, 0.13 mM of indole acrylic acid (IAA) was supplemented and culture was continued. With passage of time, 5 ml samples of the culture solution were taken and bacteria were collected by centrifugation. After washing, the cells were ground by way of ultrasonic waves and centrifuged at 15,000 r.p.m. for 15 minutes The supernatant was used as a crude enzyme solution an measured with respect to chloramphenicol acetyltransferase activity. The chloramphenicol acetyltransferase activity was measured by the method of W.V. Shaw, Methods in Enzymology, 43, 737 (1975). Similar runs were also performed on Brevibacterium lactofermentum AJ 12036 having no plasmid, the AJ 12036 strain harboring plasmid pEB 003TR having a trp promoter and a trp operator but having no trp repressor. The results are shown in Table 2. It was verified that by the addition of IAA, the chloramphenicol acetyltransferase activity was enhanced by about 5 fold with the pEC 801-harboring strain, as compared to the case where none is added, and gene expression of chloramphenicol acetyltransferase could be artificially regulated by the addition of IAA in the pEC 801-harboring strain. On the other hand, in the case of a pEB 003TR-harboring stain having no trp repressor, high activity was noted irrespective of the presence or absence of IAA and regulation of gene expression was impossible.

## Table 2

Regulation of chloramphenicol acetyltransferase gene in
Brevibacterium lactofermentum AJ 12036 using the gene-
expression regulating plasmid pEC 801

| Plasmid | IAA | Activity of Chloramphenicol Acetyltransferase | | |
| --- | --- | --- | --- | --- |
| | | 0 hr | 1 hr | 2 hr |
| None | − | 0 | 0 | 0 |
| | + | 0 | 0 | 0 |
| pEB 003TR | − | 252 | 256 | 251 |
| | + | 252 | 255 | 250 |
| pEC 801 | − | 15 | 21 | 23 |
| | + | 15 | 100 | 102 |

(unit: nmol/min. mg)

Example 5

Formation of a gene expression-regulating vector pEC 901

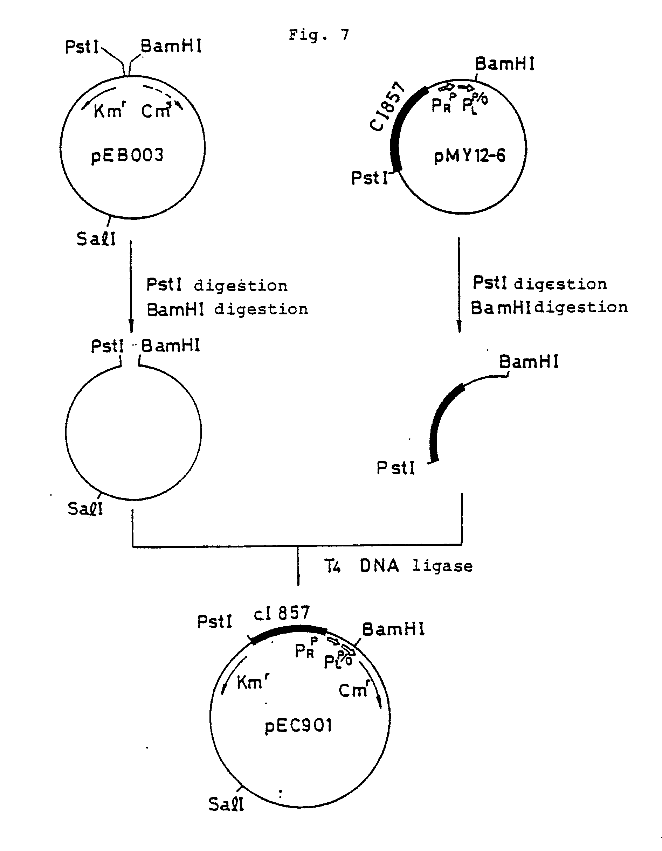

Plasmid pEC 901 capable of regulating expression of a gene ligated downstream from a $P_RP_L$ promoter and operator region of lambda phage by the action of a temperature-sensitive cI 857 repressor was formed by the method as shown in FIGURE 7. After pMY 12-6 (Mol. Gen. Genet., 187, 79-86 (1982)) was cleaved with Pst I and BamH I, DNA fragments of about 1.35 kb containing a $P_RP_L$ promoter operator and cI 857 repressor were recovered from agarose gel. On the other hand, pEP 003 was cleaved wtih Pst I and BamH I, and DNA fragments of about 7.4 kb were recovered from agarose gel. The two DNA fragments were ligated with $T_4$ DNA ligase and then introduced into Escherichia coli HB 101, and strains showing a kanamycin resistance were selected. From the separated strains, plasmid DNA of about 8.7 kb formed by ligation of the 2 DNA fragments was obtained. It was confirmed that it was the desired plasmid by cleavage patterns with restriction enzymes such as Pst I, BamH I, etc. This plasmid was named pEC 901. pEC 901 was introduced into Brevibacterium lactofermentum AJ 12036 (FERM BP-734) and Corynebacterium glutamicum ATCC 13060, from which strains those showing a kanamycin resistance were selected. From the separated strains, plasmid DNA was obtained. It was confirmed that all of the DNA was that of pEC 901 by the size of the plasmid and cleavage pattern with restriction enzymes such as Pst I, BamH I, etc.

The thus formed pEC 901 is a shuttle vector carrying a lambda phage $P_RP_L$ promoter, operator and cI 857 repressor, being capable of replicating in Escherichia coli, Brevibacterium lactofermentum and Corynebacterium glutamicum and expressing a kanamycin resistance. It is also a vector bearing a structural gene region of a chloramphenicol-resistant gene and capable of regulating expression of the chloramphenicol-resistant gene by temperature using the cI 857 repressor. Further, there are sites which may be cleaved with restriction enzymes such as BamH I, Cla I, etc. downstream from the $P_RP_L$ promoter. pEC 901 is also a vector capable of regulating expression of the gene by incorporating a structural gene region of any useful gene free from a promoter, utilizing these sites as cloning sites.

Regulation of gene expression using the gene expression-regulating vector pEC 901

Brevibacterium lactofermentum AJ 12036 (FERM-BP 734) harboring pEC 901 was inoculated on 50 ml of liquid medium of pH 7.2 containing 1% yeast extract, 1% polypeptone, 0.5% NaCl, 0.5% glucose and 10 μg/ml of kanamycin followed by shake culture at 30° C. When the $OD_{570}$ reached 0.4, the culture solution was treated at 43° C for 5 minutes followed by shake culture at 39° C. With passage of time, 20 ml samples of the culture solution were taken and bacterial cells were collected by centrifugation. After washing, the cells were ground via ultrasonic waves and centrifuged at 15,000 r.p.m. for 15 minutes. The supernatant was used as a crude enzyme solution and measured with respect to chloramphenicol acetyltransferase activity. The chloramphenicol acetyltransferase activity was measured by a method similar to Example 3. Similar runs were also performed on Brevibacterium lactofermentum AJ 12036 having pEB 003. The results are shown in Table 3. By elevating the temperature, the chloramphenicol acetyltransferase activity was expressed in the pEC 901-harboring strain and in the case where no temperature was elevated, the activity was not observed. On the other hand, no enhancement of the chloramphenicol acetyltransferase activity due to elevation of the temperature was noted in the case of the pEB 003-harboring strain.

## Table 3

Regulation of chloramphenicol acetyltransferase gene in Brevibacterium lactofermentum AJ 12036 using the gene-expression regulating plasmid pEC 901

| Plasmid | Culture Temperature | Activity of Chloramphenicol Acetyltransferase | | |
| --- | --- | --- | --- | --- |
| | | 0 hr | 6 hr | 24 hr |
| pEB 003 | 30°C | 29 | 33 | 18 |
| | 40°C | 29 | 17 | 17 |
| pEC 901 | 30°C | 0 | 0 | 0 |
| | 40°C | 0 | 80 | 103 |

(unit: nmol/min. mg)

Example 6

Regulation of gene expression in the presence of more than one plasmid

With respect to a method for regulating gene expression by incorporating an operator and a gene coding for a repressor protein in separate plasmids and having them co-exist in the same host cell, an example using a trp operator and a trp repressor is shown below. However, the combination of operator and repressor is not limited to the combination of a trp operator and a trp repressor shown in this example but may be any combination of a lac operator and a lac repressor, lambda phage, $P_RP_L$ operator and lambda phage repressor (cl 857 repressor, etc.), or the like.

As a plasmid bearing a trp promoter, trp operator and chloramphenicol acetyltransferase gene, pEB 003TR formed in Example 4 was used. In the case where pEB 003TR alone was introduced into Brevibacterium lactofermentum AJ 12036 (FERM BP-734), it was impossible to regulate the chloramphenicol acetyltransferase activity, as shown in Example 4.

As the plasmid bearing a trp repressor gene, any repressor can be used as long as it can co-exist with pEB 003TR in Coryneform bacterial cells and produces a trp repressor. For example, pEC 830 obtained by incorporating a trp repressor gene into trimethoprim-resistant plasmid vector pAJ 228, and the like, can be used.

Formation of plasmid vector pAJ 228

(1) Trimethoprim-resistant variant AJ 12146 (FERM-P 7672) derived from mutation of Brevibacterium lactofermentum AJ 12036 was inoculated on a 1 liter CM2G medium (1 g/dl of peptone, 1 g/dl of yeast extract, 0.5 g/dl of glucose and 0.5 g/dl of NaCl, adjusted pH to 7.2) followed by shake culture at 30°C for about 3 hours. Bacterial cells were collected at the exponential growth phase. After the cells were lysed with lysozyme-SDS, chromosomal DNA was extracted and purified by conventional treatment with phenol to finally obtain 3.0 mg of DNA.

Brevibacterium lactofermentum AJ 12146 was obtained by contacting Brevibacterium lactofermentum AJ 12036 with 1,000 μg/ml of N-methyl-N'-nitro-N-nitrosoguanidine at 0°C for 20 minutes for variation treatment and separating a strain capable of growing in minimum medium (containing 2 g/dl of glucose, 1 g/dl of ammonium sulfate, 0.25 g/dl of urea, 0.1 g/dl of KH₂PO₄, 0.04 g/dl of MgSO₄·7H₂O, 200 μg/1 of thiamine hydrochloride, 50 μg/1 of biotin, 2 ppm of iron irons, 2 ppm of manganese ions and 1.5 g/dl of agar, pH adjusted to 7.0) supplemented with 100 μg/ml of trimethoprim.

(2) As the vector, pAM 330 was used. pAM 330 was prepared as follows:

First, Brevibacterium lactofermentum ATCC 13869 having pAM 330 as plasmids was inoculated on 100 ml of CM2G medium. After culturing at 30°C to reach late exponential growth phase, the cells were lysed by lysozyme and SDS. The supernatant was obtained by ultracentrifugation of 30,000 x g for 30 minutes. After treatment with phenol, 2 volumes of ethanol were added to recover DNA as a precipitate. After the DNA was dissolved in a small quantity of TEN buffer (20 mM tris-hydrochloride, 20 mM NaCl, 1 mM EDTA, pH 8.0), the solution was subjected to agarose gel electrophoresis for separation. Then, the separated product was taken out to obtain about 15 μg of pAM 330 DNA.

(3) The chromosomal DNA, 20 μg obtained in (1) and 10 μg of the plasmid DNA obtained in (2) were treated with restriction endonuclease Mbo I at 37°C for 30 minutes, respectively, to effect partial cleavage. After heat treatment at 65°C for 10 minutes, both reaction solutions were mixed with each other, and the mixture was subjected to a ligation reaction of DNA strands with T₄ phage-derived DNA ligase at 10°C for 24 hours in the presence of ATP and dithiothreitol. After heat treatment at 65°C for 10 minutes, a 2-fold volume of ethanol was added to the reaction solution to precipitate and harvest DNA after completion of the ligation reaction.

(4) Brevibacterium lactofermentum AJ 12036 sensitive to trimethoprim was used as recipient. As the transformation method, a protoplast transformation method was used. First, the cells were cultured in 5 ml of CM2G medium to reach an early exponential growth phase. After adding 0.6 units/ml of penicillin, shake culture was conducted for an additional 1.5 hours. Cells were harvested by centifugation and washed with 0.5 ml of SMMP medium (pH 6.5) composed of 0.5 M sucrose, 20 mM maleic acid, 20 mM magnesium chloride and 3.5% Pennassay broth (Difco) and then suspended in SMMP medium containing 10 mg/ml of lysozyme. The suspension was treated at 30°C for 20 hours to obtain protoplasts. After centrifuging at 6000 x g for 10 minutes, the protoplasts were washed with SMMP and resuspended in 0.5 ml of SMMP. The thus obtained protoplasts were mixed with 10 μg of DNA prepared in (3) in the presence of 5 mM EDTA. After polyethylene glycol was added to the mixture to reach the final concentration of 30%, the mixture was allowed to stand at room temperature for 2 minutes to incorporate DNA into the protoplasts. After the protoplasts were washed with 1 ml of SMMP medium, they were resuspended in 1 ml of SMMP, and the suspension was cultured at 30°C for 2 hours for phenotypic expression. The culture solution was spread over a protoplast renaturation medium of pH 7.0. The renaturation medium contained, per one liter of distilled water, 12 g of tris(hydroxymethyl)aminomethane, 0.5 g of KCl, 10 g of glucose, 8.1 g of MgCl₂·6H₂O, 2.2 g of CaCl₂.2H₂O, 4 g of peptone, 4 g of yeast extract powder, 1 g of Casamino Acid (Difco), 0.2 g of K₂HPO₄, 135 g of sodium succinate, 8 g of agar and 25 μg/ml of trimethoprim (Sigma Co., Ltd.).

After culturing at 30°C for 1 week, about 100 colonies appeared, which were transferred to minimum medium plates which contained 2% glucose, 1% ammonium sulfate, 0.25% urea, 0.1% KH₂PO₂, 0.04% MgSO₄.7H₂O, 2 ppm of iron ions, 2 ppm of manganese ions, 200 μg/1 thiamine hydrochloride and 50 μg/1 biotin, pH 7.0, 1.8% agar and 50 μg/ml trimethoprim) by the replica plating method to obtain a strain resistant to trimethoprim.

(5) From the strain, a lysate was prepared by the method described in (2). When plasmid DNA was detected by agarose gel electrophoresis, a plasmid obviously larger than vector pAM 330 was detected. This strain was named AJ 12147 (FERM-P 7673).

(6) To confirm that the trimethoprim-resistant genes were present on plasmids (pAJ 228) possessed by AJ 12147, Brevibacterium lactofermentum AJ 12036 was again transformed using the plasmid DNA.

Among colonies which appeared and which had trimethoprim-resistance, 10 colonies each were chosen and collected and the plasmid DNA was detected by agarose gel electrophoresis. In all of them, plasmids having the same size as that of pAJ 228 were present. It became clear that genes expressing trimethoprim resistance were present on the recombinant plasmids described above.

(7) Properties of pAJ 228 DNA

(a) The molecular weight of pAJ 228 was determined by agarose gel electrophoresis. Agarose gel electrophoresis was performed in accordance with the method of P.A. Sharp, et al. (Biochemistry, 12, 3055 (1973)) using 0.8% gel at a constant voltage of 5 V per cm of gel length for 15 hours. The molecular weight was calculated by comparing mobility with that of a molecular weight marker having a known molecular weight: λ phage Hind III fragment (BRL Co., Ltd.) subjected to reaction with 0.5 units of restriction enzyme Cla I for cleaving 1 portion of pAJ 228 with 0.5 μg of pAJ 228 at 37°C for 1 hour, which was determined to be 7.6 kb.

(b) Preparation of a restriction map of pAJ 228 DNA

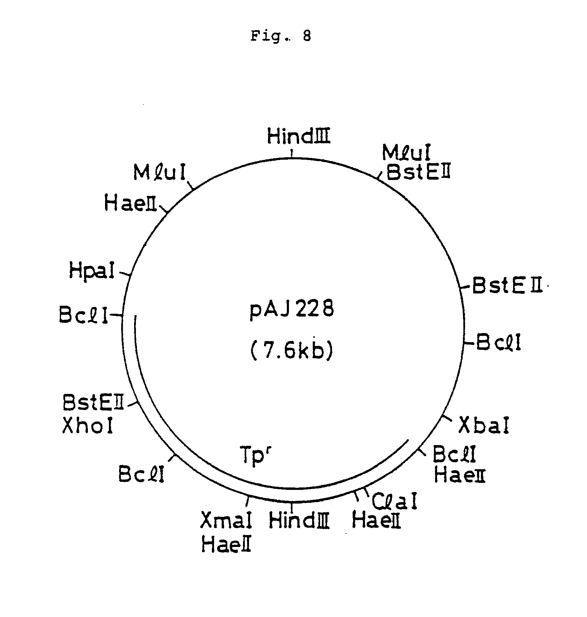

A commercially available restriction enzyme from BRL was used. Cleavage of pAJ 228 DNA with the restriction enzyme was carried out using at least a 3 fold excess of enzyme under given conditions with respect to each enzyme. In the case where plasmid DNA was cleaved with one or more restriction enzymes for the purpose of a restriction map, fragments cleaved with a first restriction enzyme were isolated by agarose gel for separation in accordance with the method of Tanaka et al. (T. Tanaka and B. Weisblum, J. Bacteriol., 121, 354 (1975)), condensed by ethanol precipitation and then cleaved with a second restriction enzyme. The cleaved fragments were subjected to agarose gel electrophoresis and their molecular weights were calculated to prepare a restriction map (FIGURE 8).

Formation of a gene expression-regulating vector pEC 830

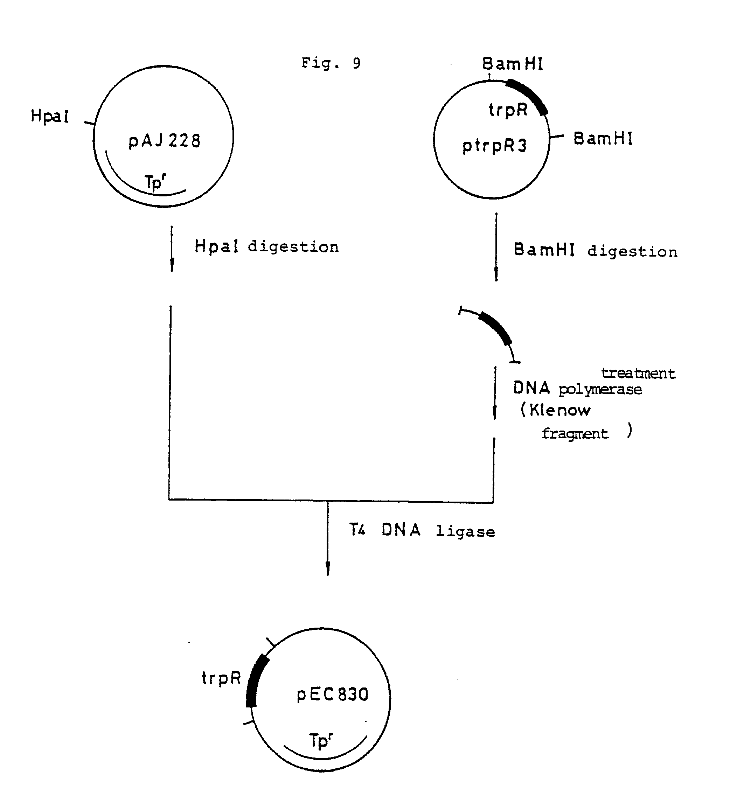

A trimethoprim-resistant vector pEC 830 for regulating gene expression having incorporated therein a trp repressor, was formed by the method shown in FIGURE 9. First, plasmid ptrp R3 was cleaved with BamH I as in Example 4, and about 1200 base pairs of DNA fragments containing a trp repressor (trp R) were recovered from agarose gel. Next, the DNA fragments were reacted with DNA polymerase (Klenow fragment) in the presence of 4 deoxynucleotides (dATP, dGTP, dCTP, dTTP) to convert the edges of the fragments into smooth terminals. The thus prepared DNA fragments and DNA of pAJ 228 cleaved with Hpa I were mixed. After ligating them using T₄ DNA ligase, they were introduced into Brevibacterium lactofermentum AJ 12036 (FERM BP-734), and strains having a trimethoprim resistance were selected. Plasmids present in the separated strains were extracted. It was confirmed that it was the desired plasmid of about 8.8 kb by the size. This plasmid was named pEC 830. pEC 830 was again introduced into Brevibacterium lactofermentum AJ 12036 (FERM BP-734) and strains having a trimethoprim resistance were selected. From the separated strains, plasmid DNA was obtained. It was confirmed that both were DNA of pEC 830 by the size of the plasmid and cleavage pattern with Xba I.

Regulation of gene expression using the gene expression-regulating vector pEC 830

pEC 830 DNA was introduced into Brevibacterium lactofermentum AJ 12036 (FERM-BP 734) harboring plasmid pEB 003TR carrying a trp promoter and a trp operator formed in Example 4. The thus obtained strain harboring both pEB 003TR and pEC 830 was inoculated on 50 ml of liquid medium containing 10 μg/ml of kanamycin and 50 μg/ml of trimethoprim and having a composition of 1% yeast extract, 1% polypeptone, 0.5% NaCl and 0.5% glucose followed by shake culture at 30°C. After culturing for 3 hours, 0.13 mM of indole acrylic acid (IAA) was supplemented and culture was continued. With passage of time, 5 ml samples of the culture solution were taken and the chloramphenicol acetyltransferase activity was measured by the method shown in Example 4. Similar runs were also performed on the AJ 12036 strain carrying pEB 003TR or pEC 830 singly and the AJ 12036 strain harboring no plasmid. The results are shown in Table 4. It was verified that by the addition of IAA, the chloramphenicol acetyltransferase activity was enhanced by about 3 to 4 fold with the strain harboring both pEB 003TR and pEC 830, as compared with the case of no addition of IAA. Thus, the gene expression could be artificially regulated by the addition of IAA. On the other hand, in the case of the strain harboring pEB 003TR singly, high activity was noted irrespective of the presence or absence of IAA, and regulation of gene expression was impossible.

## Table 4

Regulation of chloramphenicol acetyltransferase gene in Brevibacterium lactofermentum AJ 12036 using the gene-expression regulating plasmid pEC 830

| Plasmid | IAA | Activity of Chloramphenicol Acetyltransferase | | |
|---|---|---|---|---|
| | | 0 hr | 1 hr | 2 hr |
| None | − | 0 | 0 | 0 |
| | + | 0 | 0 | 0 |
| pEB 003TR | − | 210 | 225 | 218 |
| | + | 200 | 230 | 230 |
| pEC 830 | − | 0 | 0 | 0 |
| | + | 0 | 0 | 0 |
| pEB 003TR, | − | 25 | 27 | 28 |
| pEC 830 | + | 25 | 95 | 110 |

(unit: nmol/min. mg)

The invention now being fully described, it will be apparent to one of ordinary skill in the art that many changes and modifications can be made thereto without departing from the spirit or scope of the invention as set forth herein.

## Claims

1. A plasmid vector capable of replicating in a Coryneform bacterial cell, comprising a base sequence (a) functioning as a promoter in a Coryneform bacterium, a base sequence (b) functioning as an operator downstream from the base sequence (a), and being selected from a base sequence of an operator derived from Coryneform bacteria, a base sequence of a lac operator, a trp operator, a λ operator, or an operator of a phosphatase operon, a base sequence (c) functioning as a site for ribosome binding in a Coryneform bacterial cell, comprising four base pairs rich in adenine and guanine, wherein two of the upstream two base pairs are adenine, and two of the downstream two base pairs are guanine, a base sequence (d) functioning as a translation initiation codon and a gene to be expressed which is directly ligated with the base sequence (d), said vector further comprising a gene coding for a repressor protein capable of binding to said base sequence (b) functioning as an operator.

2. The plasmid vector of claim 1, wherein the distance between the base sequence (d) and the base sequence (c) is 7 to 20 base pairs.

3. The plasmid vector of claim 1, wherein said repressor protein is a repressor protein selected from the group consisting of a lac repressor, a trp repressor, a temperature-sensitive λ repressor, and a repressor of a phosphatase operon.

4. The plasmid vector pEC 701, comprising a tac promoter, a lac operator, a lac repressor, a kanamycin resistance gene, a structural gene region of a chloramphenicol-resistance gene and a cleavage site for BamH I downstream from the lac operator and having a restriction pattern as shown in fig. 4.

5. The plasmid vector pEC 702, comprising a lac promoter, a lac operator, a lac repressor, a kanamycin resistance gene, a structural gene region of a chloramphenicol-resistance gene and a cleavage site for BamH I downstream from the lac operator and having a restriction pattern as shown in fig. 5.

6. The plasmid vector pEC 801, comprising a trp promotor, a trp operator, a trp repressor, a kanamycin resistance gene, a structural gene region of a chloramphenicol-resistance gene and cleavage sites for Pst I, Hpa I and BamH I downstream from the trp promoter and having a restriction pattern as shown in fig. 6.

7. The plasmid vector pEC 901, comprising a $P_R P_L$ promoter, an operator region of a λ phage, a cI 857 repressor, a kanamycin resistance gene, a structural gene region of a chloramphenicol-resistance gene and cleavage sites for BamH I and Cla I downstream from the $P_R P_L$ promoter and having a restriction pattern as shown in fig. 7.

8. Gene expression-regulating vector pEC 830, comprising a trp repressor and a trimethoprim resistance gene and having a restriction pattern as shown in fig. 9.

9. A Coryneform bacterium containing therein a plasmid vector acc. to any of claims 1 to 8

10. A method for regulating gene expression in a host, which comprises artificially controlling binding of a repressor protein to an operator DNA sequence, said host containing a plasmid vector, wherein said plasmid vector is as defined in any of the claims 1 to 8.

11. The method of claim 10, wherein said binding of said repressor is artificially regulated by addition of chemicals or regulation of physical conditions in a medium.

12. The method of claim 11, wherein said chemical is isopropyl-β-thiogalactopyranoside

13. The method of claim 11, wherein said physical condition is temperature of the medium.

14. The method of claim 11, wherein said chemical is indole acrylic acid.

15. The method of claim 11, wherein said physical condition is phosphate ion concentration in said medium.

16. A method for regulating gene expression in a host, which comprises artificially regulating binding of a repressor protein to an operator DNA sequence, said host containing a plurality of plasmid vectors, wherein a first of said plasmid vectors comprises a base sequence (a) functioning as a promoter in a Coryneform bacterium, a base sequence (b) functioning as an operator downstream from the base sequence (a) and being selected from a base sequence of an operator derived from Coryneform bacteria, a base sequence of a lac operator, a trp operator, a λ operator, or an operator of a phosphatase operon, a base sequence (c) functioning as a site for ribosome binding in a Coryneform bacterial cell, comprising four base pairs rich in adenine and guanine, wherein two of the upstream two base pairs are adenine, and two of the downstream two base pairs are guanine, a base sequence (d) functioning as a translation initiation codon, and a gene to be expressed which is directly ligated with the base sequence (d); and a second of said plasmid vectors comprises a gene coding for a repressor protein capable of binding to the base sequence (b), said second plasmid vector being capable of co-existing with said first plasmid vector in the same host cell and being capable of replicating in a Coryneform bacterial cell.

## Revendications

1. Vecteur plasmidique capable de réplication dans une cellule de bactérie corynéforme, comprenant une séquence de bases (a) fonctionnant comme un promoteur dans une bactérie corynéforme, une séquence de bases (b) fonctionnant comme un opérateur en aval de la séquence de bases (a) et étant choisie parmi une séquence de bases d'un opérateur dérivé des bactéries corynéformes, une séquence de bases d'un opérateur lac, un opérateur trp, un opérateur de λ ou un opérateur d'un opéron phosphatase, une séquence de bases (c) fonctionnant comme un site de liaison ribosomique dans une cellule de bactérie corynéforme, comprenant quatre paires de bases riches en adénine et en guanine, où les deux paires de bases d'amont contiennent de l'adénine et les deux paires de bases d'aval contiennent de la guanine, une séquence de bases (d) fonctionnant comme un codon d'initiation de la traduction, et un gène à exprimer, qui est directement ligaturé à la séquence de bases (d), ledit vecteur comprenant de plus un gène codant pour une protéine de répression capable de se lier à ladite séquence de bases (b) fonctionnant comme un opérateur.

2. Vecteur plasmidique selon la revendication 1, dans lequel la distance entre la séquence de bases (d) et la séquence de bases (c) est de 7 à 20 paires de bases.

3. Vecteur plasmidique selon la revendication 1, dans lequel ladite protéine de répression est une protéine de répression choisie dans le groupe constitué par un répresseur lac, un répresseur trp, un répresseur thermosensible de λ et un répresseur d'un opéron phosphatase.

4. Vecteur plasmidique pEC 701, comprenant un promoteur tac, un opérateur lac, un répresseur lac, un gène de résistance à la kanamycine, une région de gène structural d'un gène de résistance au chloramphénicol et un site de clivage pour BamH I en aval de l'opérateur lac et ayant un profil de restriction comme illustré par la figure 4.

5. Vecteur plasmidique pEC 702, comprenant un promoteur lac, un opérateur lac, un répresseur lac, un gène de résistance à la kanamycine, une région de gène structural d'un gène de résistance au chloramphénicol et un site de clivage pour BamH I en aval de l'opérateur lac et ayant un profil de restriction comme illustré par la figure 5.

6. Vecteur plasmidique pEC 801, comprenant un promoteur trp, un opérateur trp, un répresseur trp, un gène de résistance à la kanamycine, une région de gène structural d'un gène de résistance au chloramphénicol et des sites de clivage pour Pst I, Hpa I et BamH I en aval du promoteur trp et ayant un profil de restriction comme illustré par la figure 6.

7. Vecteur plasmidique pEC 901, comprenant un promoteur $P_RP_L$, une région opérateur d'un phage λ, un répresseur cI 857, un gène de résistance à la kanamycine, une région de gène structural d'un gène de résistance au chloramphénicol et des sites de clivage pour BamH I et Cla I en aval du promoteur $P_RP_L$ et ayant un profil de restriction comme illustré par la figure 7.

8. Vecteur de régulation de l'expression génétique pEC 830, comprenant un répresseur trp et un gène de résistance au triméthoprime et ayant un profil de restriction comme illustré par la figure 9.

9. Bactérie corynéforme contenant un vecteur plasmidique selon l'une quelconque des revendications 1 à 8.

10. Procédé pour la régulation de l'expression génétique chez un hôte, qui comprend la régulation artificielle de la liaison d'une protéine de répression à une séquence d'ADN opérateur, ledit hôte contenant un vecteur plasmidique, où ledit vecteur plasmidique est comme défini dans l'une quelconque des revendications 1 à 8.

11. Procédé selon la revendication 10, dans lequel ladite liaison dudit répresseur est régulée artificiellement par addition d'agents chimiques ou régulation des conditions physiques dans un milieu.

12. Procédé selon la revendication 11, dans lequel ledit agent chimique est l'isopropyl-β-thiogalactopyrannoside.

13. Procédé selon la revendication 11, dans lequel ladite condition physique est la température du milieu.

14. Procédé selon la revendication 11, dans lequel ledit agent chimique est l'acide indole-acrylique.

15. Procédé selon la revendication 11, dans lequel ladite condition physique est la concentration des ions phosphates dans ledit milieu.

16. Procédé pour la régulation de l'expression génétique chez un hôte, qui comprend la régulation artificielle de la liaison d'une protéine de répression à une séquence d'ADN opérateur, ledit hôte contenant plusieurs vecteurs plasmidiques, dans lequel un premier desdits vecteurs plasmidiques comprend une séquence de bases (a) fonctionnant comme un promoteur dans une bactérie corynéforme, une séquence de bases (b) fonctionnant comme un opérateur en aval de la séquence de bases (a) et étant choisie parmi une séquence de bases d'un opérateur dérivé de bactéries coryneformes, une séquence de bases d'un opérateur lac, un opérateur trp, un opérateur de λ ou un opérateur d'un opéron phosphatase, une séquence de bases (c) fonctionnant comme un site de liaison ribosomique dans une cellule de bactérie coryneforme, comprenant quatre paires de bases riches en adénine et en guanine, où les deux paires de bases d'amont contiennent de l'adénine et les deux paires de bases d'aval contiennent de la guanine, une séquence de bases (d) fonctionnant comme un codon d'initiation de la traduction, et un gène à exprimer qui est directement ligaturé à la séquence de bases (d) ; et un second desdits vecteurs plasmidiques comprend un gène codant pour une protéine de répression capable de liaison à la séquence de bases (b), ledit second vecteur plasmidique étant capable de coexister avec ledit premier vecteur plasmidique dans la même cellule hôte et étant capable de réplication dans une cellule bactérienne coryneforme.

## Patentansprüche

1. Ein Plasmidvektor, der zur Replikation in einer coryneformen Bakterienzelle fähig ist, umfassend eine Basensequenz (a), die in einem coryneformen Bakterium als Promotor fungiert, eine Basensequenz (b), die als Operator abwärts der Basensequenz (a) fungiert und ausgewählt ist aus einer Basensequenz eines von coryneformen Bakterien stammenden Operators, einer Basensequenz eines lac-Operators, eines trp-Operators, eines λ-Operators oder eines Operators des Phosphatase-Operons, eine Basensequenz (c), die als ribosomale Bindungsstelle in einer coryneformen Bakterienzelle fungiert und vier adenin- und guaninreiche Basenpaare umfaßt, wobei zwei der aufwärts gelegenen zwei Basenpaare Adenin sind und zwei der abwärts gelegenen zwei Basenpaare Guanin sind, eine Basensequenz (d), die als Initiationskodon für die Translation fungiert sowie ein zu exprimierendes Gen, das direkt mit der Basensequenz (d) verbunden ist, wobei dieser Vektor weiterhin ein Gen umfaßt, das für ein Repressorprotein kodiert, welches zur Bindung an die als Operator fungierende Basensequenz (b) befähigt ist.

2. Der Plasmidvektor gemäß Anspruch 1, wobei der Abstand zwischen der Basensequenz (d) und der Basensequenz (c) 7 bis 20 Basenpaare beträgt.

3. Der Plasmidvektor gemäß Anspruch 1, wobei das Repressorprotein ein Repressorprotein ist, das ausgewählt ist aus der Gruppe, die aus einem lac-Repressor, einem trp-Repressor, einem temperaturempfindlichen λ-Repressor und einem Repressor des Phosphatase-Operons besteht.

4. Der Plasmidvektor pEC 701, umfassend einen tac-Promotor, einen lac-Operator, einen lac-Repressor, ein Kanamycin-Resistenzgen, eine Strukturgenregion eines Chloramphenicol-Resistenzgens und eine Spaltstelle für BamH I abwärts des lac-Operators, mit dem Restriktionsmuster, das in Fig. 4 gezeigt wird.

5. Der Plasmidvektor pEC 702, umfassend einen lac-Promotor, einen lac-Operator, einen lac-Repressor, ein Kanamycin-Resistenzgen, eine Strukturgenregion eines Chloramphenicol-Resistenzgens und eine Spaltstelle für BamH I abwärts des lac-Operators, mit dem Restriktionsmuster, das in Fig. 5 gezeigt wird.

6. Der Plasmidvektor pEC 801, umfassend einen trp-Promotor, einen trp-Operator, einen trp-Repressor, ein Kanamycin-Resistenzgen, eine Strukturgenregion eines Chloramphenicol-Resistenzgens und Spaltstellen für Pst I, Hpa I und BamH I abwärts des trp-Promotors, mit einem Restriktionsmuster, wie es in Fig. 6 gezeigt wird.

7. Der Plasmidvektor pEC 901, umfassend einen $P_RP_L$-Promotor, eine Operatorregion eines λ-Phagen, einen cI 857-Repressor, ein Kanamycin-Resistenzgen, eine Strukturgenregion eines Chloramphenicol-Resistenzgens und Spaltstellen für BamH I und CLa I abwärts des $P_RP_L$-Promotors, mit einem Restriktionsmuster, wie es in Fig. 7 gezeigt wird.

8. Ein die Genexpression regulierender Vektor pEC 830, umfassend einen trp-Repressor und ein Trimethoprim-Resistenzgen, mit einem Restriktionsmuster, wie es in Fig. 9 gezeigt wird.

9. Ein coryneformes Bakterium, das einen Plasmidvektor gemäß einem der Ansprüche 1 bis 8 enthält.

**10.** Eine Methode zur Regulation der Genexpression in einem Wirt, die die artifizielle Kontrolle der Bindung eines Repressorproteins an eine Operator-DNS Sequenz umfaßt, wobei dieser Wirt einen Plasmidvektor enthält, und dieser Plasmidvektor definiert ist wie in einem der Ansprüche 1 bis 8.

**11.** Methode nach Anspruch 10, wobei die Bindung des Repressors artifiziell reguliert wird durch Zugabe von chemischen Verbindungen oder durch Regulation der physikalischen Bedingungen in einem Medium.

**12.** Methode nach Anspruch 11, wobei die chemische Verbindung Isopropyl-$\beta$-thiogalactopyranosid ist.

**13.** Methode nach Anspruch 11, wobei die physikalische Bedingung die Temperatur des Mediums ist.

**14.** Methode nach Anspruch 11, wobei die chemische Verbindung Indolacrylsäure ist.

**15.** Methode nach Anspruch 11, wobei die physikalische Bedingung die Phosphationenkonzentration in diesem Medium ist.

**16.** Eine Methode zur Regulation der Genexpression in einem Wirt, die die artifizielle Regulation der Bindung eines Repressorproteins an eine Operator-DNS Sequenz umfaßt, wobei der Wirt eine Vielzahl von Plasmidvektoren enthält, und wobei ein erster dieser Plasmidvektoren eine Basensequenz (a), die als Promotor in einem coryneformen Bakterium fungiert, eine Basensequenz (b), die als Operator abwärts der Basensequenz (a) fungiert und ausgewählt ist aus einer Basensequenz eines von coryneformen Bakterien stammenden Operators, von einer Basensequenz des lac-Operators, eines trp-Operators, eines λ-Operators, oder eines Operators des Phosphatase-Operons, eine Basensequenz (c), die als ribosomale Bindungsstelle in einer coryneformen Bakterienzelle fungiert, und die vier adeninund guaninreiche Basenpaare enthält, wobei zwei der beiden aufwärts gelegenen Basenpaare Adenin sind und zwei der beiden abwärts gelegenen Basenpaare Guanin sind, eine Basensequenz (d), die als Initiationskodon für die Translation fungiert, und ein Gen, das exprimiert werden soll und direkt an die Basensequenz (d) gebunden ist, umfaßt; und ein zweiter dieser Plasmidvektoren ein Gen umfaßt, das für ein Repressorprotein kodiert, das zur Bindung an die Basensequenz (b) fähig ist, wobei dieser zweite Plasmidvektor befähigt ist, mit dem ersten Plasmidvektor in derselben Wirtszelle zusammen zu existieren und in einer coryneformen Bakterienzelle zu replizieren.

Fig. 1

Fig. 2

Fig. 3

```
            G T T  A A C G G A T C C A T
   A C G T C A A  T T G C C T A G G T A G C
PstI              HpaI              BamHI        ClaI
```

Fig. 4

Fig. 5

Fig. 6

EP 0 215 388 B1

Fig. 7

25

Fig. 8

Fig. 9